# EUROPEAN PATENT SPECIFICATION

(11) **EP 1 545 637 B1**
(45) Date of publication and mention of the grant of the patent: **02.08.2006**
(21) Application number: 03756550.4
(22) Date of filing: 01.10.2003
(51) Int. Cl.: A61L 15/32, A61L 15/46

(54) **ENZYME-SENSITIVE THERAPEUTIC WOUND DRESSINGS**
ENZYMABHÄNGIGE THERAPEUTISCHE WUNDVERBÄNDE
PANSEMENTS THERAPEUTIQUES SENSIBLES AUX ENZYMES

(30) Priority: 01.10.2002 GB 0222722
(43) Date of publication of application: 29.06.2005
(73) Proprietor: Johnson & Johnson Medical Ltd., Edinburgh EH2 4NH (GB)
(72) Inventor: TROTTER, Patrick, Leeds LS6 4RD (GB); SILCOCK, Derek, North Yorkshire BD23 1QP (GB)
(74) Representative: James, Anthony Christopher W.P.
(86) International application number: PCT/GB2003/004250
(87) International publication number: WO 2004/030711

(56) References cited:
- WO-A-00/08203
- US-A- 5 098 417
- US-A- 5 147 339

## Description

The present invention relates to wound dressing materials, and in particular to new materials for the controlled release of therapeutic agents into wounds.

In mammals, injury triggers an organised complex cascade of cellular and biochemical events that result in a healed wound. Wound healing is a complex dynamic process that results in the restoration of anatomic continuity and function; an ideally healed wound is one that has returned to normal anatomic structure, function and appearance.

Infection of wounds by bacteria delays the healing process, since bacteria compete for nutrients and oxygen with macrophages and fibroblasts, whose activities are essential for the healing of the wound. Infection results when bacteria achieve dominance over the systemic and local factors of host resistance. Infection is therefore a manifestation of disturbed host/bacteria equilibrium in favour of the invading bacteria. This elicits a systemic septic response, and also inhibits the multiple processes involved in wound healing. Lastly, infection can result in a prolonged inflammatory phase and thus slow healing, or may cause further necrosis of the wound. The granulation phase of the healing process will begin only after the infection has subsided.

Chronically contaminated wounds all contain tissue bacterial flora. These bacteria may be indigenous to the patient or might be exogenous to the wound. Closure or eventual healing of the wound is often based on a physician's ability to control the level of the bacterial flora.

If clinicians could respond to wound infection as early as possible the infection could be treated topically as opposed to having to use antibiotics. This would also lead to less clinical intervention/hospitalisation and would reduce the use of antibiotics and other complications of infection.

Current methods used to identify bacterial infection rely mainly on judgement of the odour and appearance of a wound. With experience, it is possible to identify an infection in a wound by certain chemical signs such as redness or pain. Some clinicians take swabs that are then cultured in the laboratory to identify specific organisms, but this technique takes time.

Pain is also associated with infected and chronic wounds. Biochemically, pain is experienced when there is an increase of kinins (bradykinin) in the area of the wound. Kinins are produced by the proteolytic breakdown of kininogen, and the protease responsible for this is kallikrein. Kallikrein also stimulates the production of tissue plasminogen activator (t-PA).

It has now been discovered that wound fluid from wounds that are apparently not clinically infected but which go on to become infected within a few days have elevated levels of neutrophil elastase activity and may also have high levels of other inflammatory enzymes, such as macrophage proteases, other neutrophil proteases, bacterial collagenase, plasmin, hyaluronidase, kallikrein or t-PA.

Further, chronic wounds, such as venous ulcers, pressure sores and diabetic ulcers have a disordered wound-healing metabolism even in the absence of infection. In particular, wound chronicity is associated with elevated levels of protease enzymes in the wound that interfere with the normal processes of tissue formation and destruction in the wound.

It is known to provide antimicrobial wound dressings. For example, such dressings are known having a liquid permeable wound contacting layer, an intermediate absorbent layer and an outer, liquid-impervious backing layer, in which one or more of the layers contains an antimicrobial agent. For example, EP-A-0599589 describes layered wound dressings having a wound contacting layer of a macromolecular hydrocolloid, an absorbent layer, and a continuous, microporous sheet intermediate the wound contacting layer and the absorbent layer. The absorbent layer contains a low molecular weight antimicrobial agent that can diffuse into the wound.

WO-A-0238097 describes wound dressings comprising a liquid-permeable top sheet having a wound facing surface and a back surface, and a hydrogel layer on the wound-facing surface of the top sheet. The top sheet is adapted to block or restrict passage of liquid from the back surface to the wound-facing surface. The hydrogel layer is an insoluble hydrogel adapted to maintain a moist wound-healing environment at the wound surface. The hydrogel may contain therapeutic agents, such as antimicrobial agents, for sustained release into the wound.

Previous antimicrobial wound dressings suffer from the drawback that the release of the antimicrobial agent is relatively unresponsive to the degree of infection of the wound being treated. This is undesirable because it can result in resistant microorganisms, and also because all unnecessary medication can interfere with the processes of wound healing.

A first aspect of the invention provides a wound dressing comprising a therapeutic agent and a matrix comprising polymers joined by cross-linkages which cross-linkages comprise, or consist of, oligopeptidic sequences which are cleavable by a protease associated with wound fluid such that the rate of release of the therapeutic agent increases in the presence of the protease.

Preferably, the matrix consists of the cross-linked polymers and optionally also the therapeutic agent.

Preferably, the protease is associated with a wound healing disorder, e.g. infection, or ulcer formation (wound chronicity). In this way, the rate of release of the therapeutic agent may increase if the wound is infected or is a chronic wound.

By an "increase" in the rate of release of the therapeutic agent we include the situation where the rate of release of the therapeutic agent increases by at least 1.5, 2-, 3-, 4-, 5-, 6-, 7-, 8-, 9-, 10- or 15-fold. Preferably, there is no release of the therapeutic agent in the absence of the protease.

By "a protease associated with wound infection" we include proteases that are elevated during infection and proteases that are elevated in wounds that are apparently not clinically infected but which go on to become infected within a few days. Similarly, by "a protease associated with ulcer formation" we include proteases that are elevated in chronic wounds.

The principle underlying the present invention is that the cross-linked polymers would behave as both an enzyme sensor and as an enzyme-dependent delivery system. In the absence of the target protease the oligopeptidic sequences remain intact, keeping the pore size small and preventing (or at least keeping to low levels) the release of the therapeutic agent. Elevated protease levels (e.g. in wound infection or wound chronicity) hydrolyse the oligopeptidic sequences which results in increased pore size and permeability. The therapeutic agent is then released from the dressing so that it is free to migrate into the wound. In this way, delivery of the therapeutic agent increases in the presence of the protease so that if the wound is infected (including as indicated above when a protease associated with infection is elevated in a wound that is apparently not clinically infected but which goes on to become infected within a few days) or is a chronic wound delivery of the therapeutic agent increases.

The term "polymer" as used herein includes homopolymers and copolymers (e.g. random copolymers, alternating copolymers and block copolymers).

Although polymers which are degraded by the target protease could be used, it is preferred that the polymers are not degraded by the target protease or other factors (e.g. other proteases) that may be present in the wound environment.

In theory, any polymer containing groups to which the reactive groups can be attached may be used, although of course the skilled person will appreciate that considerations such as toxicity should be taken into account. Similarly, the polymers used should not be immunogenic.

In selecting a polymer, charge and size may also be important as an increase in crystallinity will increase order and therefore reduce permeability of barrier. The longer the polymers the more likely they are to become physically intertwined, and consequently the less likely they are to fall apart. In view of this it is preferred that short polymers (i.e. 5 to 50 monomers) are used.

Preferably, a polyfunctional polymer is used as the pore size will be smaller and the ability to retain the therapeutic agent in the absence of protease will be higher.

Preferably, the polymers are non-ionic surfactants, polyalkoylated alcohols, alkyl or dialkyl polyglycerol compounds, polyethyloxylated alcohols, polymers (including homopolymers and copolymers) of acrylamide (e.g. N-(2-hydroxypropyl)methacrylamide (HPMA)), polynucleotides, polypeptides or carbohydrates.

Preferably, the polymers are synthetic polymers. Examples of synthetic polymers include polyvinyl alcohol, polyethylene glycerol, PVP, polyolefins, fluoropolymers, hydropolymers from vinyl esters, vinyl ethers, carboxy vinyl monomers, meth(acrylic) acid, acrylamide, N-vinyl pyrrolidone, acylamidopropanem acylamidopropane , PLURONIC (Maleic acid, NN-dimethylacrylamide diacetone acrylamide acryloyl, morpholine and mixtures thereof, and oxidized regenerated cellulose.

Alternatively, natural polymers such as carbohydrates (e.g. dextan, chitin or chitosan) natural peptides or proteins (collagens, elastin, fibronectins, or even soluble proteins such as albumin), or semi synthetic peptides (made by using a peptide synthesizer or by recombinant techniques) may be used.

In a preferred embodiment, polymers of N-(2-hydroxypropyl) methyacrylamide (HPMA) are used. In this regard, reference is made to Ulbrich *et al.* (1980) *Biomaterials* 1, 199-204, which details the crosslinking of HPMA polymers by peptides.

As mentioned above, the polymers are joined by cross-linkages which comprise cleavable oligopeptidic sequences. Oligopeptides are generally defined as polypeptides of short length, typically twenty amino acids or fewer. Preferably, the oligopeptidic sequences employed in the present invention consist of 3 to 15 amino acids, preferably 3 to 10 amino acids, more preferably, 3 to 8 amino acids and yet more preferably 4 to 8 amino acids. Preferably, the oligopeptidic sequences consist of 3, 4, 5, 6, 7 or 8 amino acids.

The degree of crosslinking of the polymers should be sufficient such that the rate of release of the therapeutic agent increases in the presence of the protease. Preferably, the degree of crosslinking of the polymers should be sufficient to render the matrix sufficiently impermeable to the molecule to be delivered so that the therapeutic agent is only released in the presence of the target protease. This will be dependent on the molecular weight of the therapeutic agent.

The rate of degradation of the matrix will depend on a number of factors, including the length of the oligopeptidic sequences. Ulbrich *et al.* noted that extension of the peptidic linkers by one amino acid residue to give a peptidic linker of four amino acids caused a pronounced rise in the rate of cleavage of the polymeric substrates. Ulbrich et al. reported that extension of the oligopeptidic sequence led to a decrease in the steric hindrance by polymer chain and thus to an increase in degradability.

Steric hindrance may also be reduced by coupling the oligopeptidic sequence to the polymer by means of an appropriate spacer. Thus, the oligopeptidic sequences may couple the polymers directly (in which case the cross-linkage consists of the oligopeptidic sequence) or by means of an appropriate spacer.

The following paper gives a useful review of bioconjugation techniques for use in pharmaceutical chemistry: Veronese, F.M. and Morpurgo, M (1999) Bioconjugation in Pharmaceutical chemistry II Farmaco, 54, 497-516. This paper describes in detail the chemistry of each amino acid and which ones are most suitable for use in bioconjugation techniques. For example, it demonstrates that conjugation would occur by nucleophile to electrophile attacks. The amino acid side chains R-S-, R-NH2, R-COO- and =R-O- are well suited to bioconjugation (to natural or synthetic molecules).

In addition this paper indicates and gives examples of a wide range of structures and chemical groups that the peptides (containing amino (e.g. lysine), carboxyl (COO-) or cystyl groups (R-SH) can bind to.

With regard to conjugation techniques, see also Ulbrich, K., *et al* (2000) Polymeric drugs based on conjugates of synthetic and natural marcomolecules I. Synthesis and physico-chemical characterisation. Journal of controlled release 64, 63-79. This reference describes how antibodies, peptides or proteins can be conjugated to synthetic polymers (e.g. poly HPMA).

The rate of degradation will not only depend on the number of amino acids but also on the nature of the amino acids comprising the cross-links. This dependency arises from the substrate specific nature of proteases. The region of the enzyme where interaction with the substrate takes place is known as the "active site" of the enzyme. The active site performs the dual role of binding the substrate while catalysing the reaction, for example cleavage. Studies of the structures of the complexes of proteolytic enzymes with peptides indicate that the active site of these enzymes is relatively large and binds to several amino acid residues in the peptide. Thus, the degradability of a particular bond in a peptide chain depends not only on the nature of the structure near the cleaved bond, but also on the nature of the amino acid residues which are relatively remote from the cleaved bond, but play an important part in holding the enzyme in position during hydrolysis.

The structure of the oligopeptidic sequences must be chosen so as to correspond to that of the active site of the protease responsible for the degradation. The protease may be a host-derived protease or a protease produced by pathogens (e.g. bacteria) at the site of infection. Examples of such enzymes include, but are not restricted to: matrix metalloproteinases and other extracellular matrix component proteases (including collagenases, stromelysins, matrilysin, gelatinases and elastases), lysosomal enzymes (including cathepsin), serine proteases and other enzymes of the clotting cascade (such as thrombin), enzymes of the endoplasmic reticulum (such as cytochrome P450 enzymes, hydrolytic reaction enzymes and conjugation reaction enzymes), non-specific aminopeptidases and esterases, carboxypeptidases, phosphatases, and glycolytic enzymes. Thrombin-like, alanine aminopeptidase, and elastase-like enzymatic activity are common in bacterial infections, and the amino acid cleavage sequences of such enzymes are well-documented. WO 00/64486 discloses amino acid cleavage sequences of various enzymes associated with wound infection.

Preferably, the protease is a macrophage or neutrophil protease, or a human or bacterial collagenase or gelatinase. The macrophage and neutrophil proteases include elastase, matrix metalloproteinase 9 (MMP-9), MMP-8, cathepsin G, MMP-12, capases and mixtures thereof.

Preferably, the protease is a collagenase, gelatinase, elastase, matrix metalloproteinase, stromelysin, cathepsin G, thrombin or capase.

In one embodiment, the protease is elastase and the oligopeptidic sequence comprises or consists of lys-gly-ala-ala-ala-lys, -Ala-Ala-Ala-, Ala-Ala-Pro-Val, Ala-Ala-Pro-Leu, Ala-Ala-Pro-Phe, Ala-Ala-Pro-Ala or Ala-Tyr-Leu-Val.

Preferably, the oligopeptidic sequence is cleavable by elastase but is not cleavable by a MMP such as MMP-2 or MMP-9.

In another embodiment, the protease is a matrix metalloproteinase and the oligopeptidic sequence comprises or consists of -Gly-Pro-Y-Gly-Pro-Z-, -Gly-Pro-Leu-Gly-Pro-Z-, -Gly-Pro-Ile-Gly-Pro-Z-, or-Ala-Pro-Gly-Leu-Z-, where Y and Z are amino acids where Y and Z are amino acids.

In another embodiment, the protease is a collagenase and the oligopeptidic sequence comprises or consists of -Pro-Leu-Gly-Pro-D-Arg-Z-, -ProLeu-Gly-Leu-Leu-Gly-Z-, -Pro-Gln-Gly-Ile-Ala-Gly-Trp-, -Pro-Leu-Gly-Cys(Me)-His-, -Pro-Leu-Gly-Leu-Trp-Ala-, -Pro-Leu-Ala-Leu-Trp-Ala-Arg-, or-Pro-Leu-Ala-Tyr-Trp-Ala-Arg-, where Z is an amino acid.

In another embodiment, the protease is a gelatinase and the oligopeptidic sequence comprises or consists of -Pro-LeuGly-Met-Trp-Ser-Arg-.

In another embodiment, the protease is a thrombin and the oligopeptidic sequence comprises or consists of -Gly-Arg-Gly-Asp-,-Gly-Gly-Arg-,-Gly-Arg-Gly-Asp-Asn-Pro-,-Gly-Arg-Gly-Asp-Ser-,-Gly-Arg-Gly-Asp-Ser-Pro-Lys-,-Gly-Pro-Arg-,-Val-Pro-Arg-, or-Phe-Val-Arg-.

In another embodiment, the protease is a stromelysin and the oligopeptidic sequence comprises or consists of -Pro-TyrAla-Tyr-Trp-Met-Arg-.

In one preferred embodiment of the invention, the protease is elastase and the polymers are HPMA polymers and the oligopeptidic sequences comprise or consist of lys-gly-ala-ala-ala-lys, -Ala-Ala-Ala-, Ala-Ala-Pro-Val, Ala-Ala-Pro-Leu, Ala-Ala-Pro-Phe, Ala-Ala-Pro-Ala or Ala-Tyr-Leu-Val. Preferably, the oligopeptidic sequences directly couple the HPMA polymers (i.e. without the presence of spacers). In this example the terminal lysines may be added to bind the peptide to the polymer.

Preferably, the oligopeptidic sequences are cleavable by only one protease associated with wound fluid, preferably elastase. Alternatively, the oligopeptidic sequences may be cleavable by two, three or more proteases associated with wound fluid.

The design of the linking oligopeptidic sequence is important as it must not only contain a hydrolysable sequence that would be cleaved in the presence of the protease but also a terminal amino acid that can be readily conjugated to the polymers employed or to a spacer. Examples of reactive amino acids that could be used to link the oligopeptidic sequences to the polymers or spacers include cysteine and lysine.

The therapeutic agent may, for example, be an antimicrobial agent and/or a pain relieving agent. The antimicrobial agent may, for example, comprise an antiseptic, an antibiotic, or mixtures thereof.

In selecting one or more therapeutic agents for use with the wound dressings of the present invention, it is preferred that larger molecules are employed (e.g. molecules having a molecular weight of at least 500, 1,000, 5,000, 10,000, or 20,000). Small molecules may penetrate the matrix, whereas larger molecules such as chlorohexidine may be better suited to this type of application. Bioerodible particles and colloidal silver are suitable. Although it is preferred that the therapeutic agent is one which cannot penetrate the matrix, it should nevertheless be appreciated that small molecules such as silver salts may be employed since the release of such therapeutic agents may still be to some extent responsive to levels of protease as in the presence of elevated levels of the target protease the cross-linkages which be cleaved which will result in an increase in the rate of release of the therapeutic agent. Further, if a polyfunctional polymer is used the pore size of the matrix will be smaller and thus the ability of the matrix to retain the therapeutic agent in the absence of protease will be higher. Moreover, as noted above, the degree of cross-linking will influence the permeability of the matrix.

Preferred antibiotics include peptide antimicrobials (e.g. defensins, Magainin, synthetic derivatives of them) tetracycline, penicillins, terramycins, erythromycin, bacitracin, neomycin, polymycin B, mupirocin, clindamycin and mixtures thereof. Preferred antiseptics include silver sulfadiazine, chlorhexidine, povidone iodine, triclosan, other silver salts, sucralfate, quaternary ammonium salts and mixtures thereof. The pain relieving agent may be an analgesic or a local anaesthetic.

The therapeutic agent may be incorporated within the matrix of the invention or may alternatively be located behind the matrix in a "donor layer". Thus, in one embodiment of the first aspect invention, the therapeutic agent is incorporated within the matrix. For ready release of the therapeutic agent upon elevation of the target protease, the therapeutic agent should not covalently bound to the matrix. For example, if the molecule to be delivered is relatively inert it could be mixed into the formulation during manufacture. Silver is one example of a molecule that could be delivered in this way. The wound contacting layer of the dressing may comprise or consist of the matrix in which the therapeutic agent has been incorporated into. Alternatively, the dressing may comprise a liquid permeable wound contacting layer, an intermediate layer (which may be an absorbent layer) comprising or consisting of the matrix within which the therapeutic agent has been incorporated and an outer, liquid-impervious backing layer. Upon degradation of the matrix by proteases present in wound fluid, the therapeutic agent present in the intermediate layer may diffuse into the wound.

Another embodiment of the first aspect of the invention provides a wound dressing which comprises a barrier layer which comprises the cross-linked matrix of the invention, the barrier layer being for initially separating the therapeutic agent in the wound dressing from wound fluid when in use. Suitably, the barrier layer consists of the matrix.

The barrier layer is separate from the therapeutic agent, and the therapeutic agent is initially prevented from contacting the wound fluid by the barrier layer. That is to say, the bioavailability of the therapeutic agent to the wound surface is low until the peptide cross-linkages in the barrier material have been broken down by the enzyme, at which point the bioavailability of the therapeutic agent increases. Since protease levels are elevated in chronic and infected wounds, this provides for accelerated and/or selective release of the therapeutic agent into such wounds. The barrier layer is normally substantially impervious to wound fluid and insoluble therein unless the wound fluid contains a sufficient level of the specified enzyme to break down the substrate material.

The barrier layer is preferably about 0.1 to about 3 mm thick. Preferably about 0.5 to 1.5 mm thick. The cross-linked polymers may be combined in a film-forming composition with polymeric materials, plasticisers, and humectants. Suitable polymers include alginates, guar gum, carboxymethyl cellulose, methyl cellulose, hydroxypropyl methyl cellulose, locust bean gum, carrageenan, chitosan, heparan sulfate, dermatan sulfate, glycosaminoglycans such as hyaluronic acid, proteoglycans, and mixtures thereof. Suitable plasticisers include C2-C8 polyhydric alcohols such as glycerol. Preferably the cross-linked polymers make up at least about 10% by weight, more preferably at least about 20%, 30%, 40%, 50%, 60%, 70%, 80% or 90% by weight of the film-forming composition.

In certain embodiments the barrier layer comprises a substantially continuous film comprising the film forming composition of the cross-linked polymers as described above.

In other embodiments the barrier layer comprises an apertured sheet having a composition comprising the cross-linked polymers applied thereto in occlusive fashion. The occlusive composition may be similar to the film-forming composition described above. In these embodiments, the apertures typically make up from about 0.1% to about 50% of the area of the wound facing surface of the sheet before swelling, more typically from about 1% to about 30% of the area of the apertured sheet, and preferably from about 10% to about 25% of the area of the apertured sheet. Typically, the apertured sheet has from about 1 to about 30 apertures per square cm, for example from about 4 to about 15 apertures per square cm or from about 5 to about 10 apertures per square cm. In certain embodiments the apertures are uniformly distributed over the surface of the sheet, preferably in a regular pattern. The mean area of each aperture may for example be from about 0.01 to about 10 mm², preferably from about 0.1 to about 4 mm², and more preferably from about 1 mm² to about 2mm². It will be appreciated that the sheet may include more than one size and shape of aperture in order to provide apertures that open more or less quickly on exposure to infected wound fluid. This enables still more control over the dynamics of therapeutic agent delivery to the wound. Typically, substantially the whole area of the apertures in the apertured sheet is blocked by the barrier material before exposure to wound exudate.

Preferably, the thickness of the barrier film or the apertured sheet (by ASTM D374-79) is from about 0.2 to about 5 mm, more preferably from about 0.4 to about 3 mm.

In one embodiment the barrier layer material may comprise, in addition to the cross-linked matrix of the invention, a polymer selected from the group consisting of water soluble macromolecular materials (hydrogels) such as sodium alginate, sodium hyaluronate, alginate derivatives such as the propylene glycol alginate described in EP-A-0613692, and soluble hydropolymers formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof. Suitable hydrogels are also described in US-A-5352508.

In one embodiment the barrier layer material may comprise, in addition to the cross-linked matrix of the invention, a polymer selected from the group consisting of bioerodible polymers such as polylactide/polyglycolide, collagen, gelatin, polyacrylate gels such as those described in EP-A-0676457, calcium alginate gels, cross-linked hyaluronate gels, gels of alginate derivatives such as propylene glycol alginate, and gels wherein the hydropolymer is formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof. Suitable hydrogels are also described in US-A-5352508.

The barrier layer material may further comprise from about 5 to about 50% by weight, preferably from 15 to 40% by weight, on the same basis of one or more humectants such as glycerol. The barrier layer material may further contain up to about 30% w/w, more preferably up to about 15% w/w on the same basis of water.

The matrix of the invention comprising the therapeutic agent may contact the barrier layer directly, or may be separated therefrom for example by an absorbent layer.

Preferably, the wound dressing of the invention comprises an absorbent layer and/or a backing layer. As will be evident from the above, the absorbent layer may, for example, separate the barrier layer from the therapeutic agent containing cross-linked matrix or alternatively the absorbent layer may comprise the therapeutic agent containing cross-linked matrix.

The area of the optional absorbent layer is typically in the range of from 1 cm² to 200cm², more preferably from 4cm² to 100cm².

The optional absorbent layer may comprise any of the materials conventionally used for absorbing wound fluids, serum or blood in the wound healing art, including gauzes, nonwoven fabrics, superabsorbents, hydrogels and mixtures thereof. Preferably, the absorbent layer comprises a layer of absorbent foam, such as an open celled hydrophilic polyurethane foam prepared in accordance with EP-A-0541391. In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1 mm to 4mm. The free (uncompressed) liquid absorbency measured for physiological saline may be in the range of 5 to 30 g/g at 25°.

Preferably, the wound dressing further comprises a backing layer covering the barrier sheet and the optional absorbent layer on the side opposite the wound-facing side of the dressing. The backing layer preferably provides a barrier to passage of microorganisms through the dressing and further preferably blocks the escape of wound fluid from the dressing. The backing layer may extend beyond at least one edge of the barrier sheet (if present) and optional absorbent layer to provide an adhesive-coated margin adjacent to the said edge for adhering the dressing to a surface, such as to the skin of a patient adjacent to the wound being treated. An adhesive-coated margin may extend around all sides of the barrier sheet (if present) and optional absorbent layer, so that the dressing is a so-called island dressing. However, it is not necessary for there to be any adhesive-coated margin.

Preferably, the backing layer is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesive layer (where present) should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Preferably, the adhesive layer extends outwardly from the absorbent layer and the envelope to form an adhesive-coated margin on the backing sheet around the absorbent layer as in a conventional island dressing.

Also within the scope of the present invention are embodiments in which the cross-linked matrix material substantially encapsulates the therapeutic agent. For example, the dressing may comprise, or consist essentially of, particles such as microspheres of therapeutic agent (e.g. antimicrobial material) encapsulated in a layer comprising the cross-linked matrix material. The particles are preferably loaded with from 1 to 90 wt.%, more preferably from 3 to 50 wt.% of the therapeutic agent.

The particles may be made by any suitable technique, including comminution, coacervation, or two-phase systems for example as described in US-A-3886084. Techniques for the preparation of medicated microspheres for drug delivery are reviewed, for example, in Polymeric Nanoparticles and Microspheres, Guiot and Couvreur eds., CRC Press (1986).

A preferred method for preparation of the microparticles is coacervation, which is especially suited to the formation of particles in the preferred size range of 100 to 500 micrometers having a high loading of therapeutic agents. Coacervation is the term applied to the ability of a number of aqueous solutions of colloids, to separate into two liquid layers, one rich in colloid solute and the other poor in colloid solute. Factors which influence this liquid-liquid phase separation are: (a) the colloid concentration, (b) the solvent of the system, (c) the temperature, (d) the addition of another polyelectrolyte, and (e) the addition of a simple electrolyte to the solution. Coacervation can be of two general types. The first is called "simple" or "salt" coacervation where liquid phase separation occurs by the addition of a simple electrolyte to a colloidal solution. The second is termed "complex" coacervation where phase separation occurs by the addition of a second colloidal species to a first colloidal solution, the particles of the two dispersed colloids being oppositely charged. Generally, materials capable of exhibiting an electric charge in solution (i.e. materials which possess an ionizable group) are coacervable. Such materials include natural and synthetic macromolecular species such as gelatin, acacia, tragacanth, styrene-maleic anhydride copolymers, methyl vinyl ether-maleic anhydride copolymers, polymethacrylic acid, and the like.

If, prior to the initiation of coacervation, a water-immiscible material, such as an oil, is dispersed as minute droplets in an aqueous solution or sol or an encapsulating colloidal material, and then, a simple electrolyte, such as sodium sulfate, or another, oppositely charged colloidal species is added to induce coacervation, the encapsulating colloidal material forms around each oil droplet, thus investing each of said droplets in a liquid coating of the coacervated colloid. The liquid coatings which surround the oil droplets must thereafter be hardened by cross-linking to produce solid-walled microcapsules

Preferably, the wound dressing according to any aspect of the present invention is sterile and packaged in a microorganism-impermeable container.

## Claims

1. A wound dressing comprising a therapeutic agent and a matrix comprising polymers joined by cross-linkages which cross-linkages comprise, or consist of, oligopeptidic sequences which are cleavable by a protease associated with wound fluid such that the rate of release of the therapeutic agent increases in the presence of the protease.

2. A wound dressing according to claim 1 wherein the protease is associated with wound infection or ulcer formation.

3. A wound dressing according to claim 1 or 2 wherein the polymers themselves are not degraded by the protease or other factors that may be present in the wound environment.

4. A wound dressing according to claim 1, 2 or 3 wherein the polymer is a synthetic polymer.

5. A wound dressing according to claim 4 wherein the polymer is a polymer of N-(2-hydroxypropyl) methyacrylamide (HPMA).

6. A wound dressing according to any one of the preceding claims wherein the oligopeptidic sequences consist of 3 to 15 amino acids.

7. A wound dressing according to any one of the preceding claims wherein the protease is elastase and wherein the oligopeptidic sequence comprises or consists of lys-gly-ala-ala-ala-lys -Ala-Ala-Ala-, Ala-Ala-Pro-Val, Ala-Ala-Pro-Leu, Ala-Ala-Pro-Phe, Ala-Ala-Pro-Ala or Ala-Tyr-Leu-Val.

8. A wound dressing according to any one of claims 1 to 6 wherein the protease is a matrix metalloproteinase and wherein the oligopeptidic sequence comprises or consists of -Gly-Pro-Y-Gly-Pro-Z-,-Gly-Pro-Leu-Gly-Pro-Z-,-Gly-Pro-Ile-Gly-Pro-Z-, or-Ala-Pro-Gly-Leu-Z-, where Y and Z are amino acids.

9. A wound dressing according to any one of claims 1 to 6 wherein the protease is a collagenase and wherein the oligopeptidic sequence comprises or consists of -Pro-Leu-Gly-Pro-D-Arg-Z-,-ProLeu-Gly-Leu-Leu-Gly-Z-,-Pro-Gln-Gly-Ile-Ala-Gly-Trp-,-Pro-Leu-Gly-Cys (Me)-His-,-Pro- Leu-Gly-Leu-Trp-Ala-,-Pro-Leu-Ala-Leu-Trp-Ala-Arg-, or-Pro-Leu-Ala-Tyr-Trp-Ala-Arg-, where Z is an amino acid.

10. A wound dressing according to any one of claims 1 to 6 wherein the protease is a gelatinase and wherein the oligopeptidic sequence comprises or consists of -Pro-LeuGly-Met-Trp-Ser-Arg-.

11. A wound dressing according to any one of claims 1 to 6 wherein the protease is thrombin and wherein the oligopeptidic sequence comprises or consists of -Gly-Arg-Gly-Asp-,-Gly-Gly-Arg-,-Gly-Arg-Gly-Asp-Asn-Pro-,-Gly-Arg-Gly-Asp-Ser-,-Gly-Arg-Gly-Asp-Ser-Pro-Lys-,-Gly-Pro-Arg-,-Val-Pro-Arg-, or-Phe-Val-Arg-.

12. A wound dressing according to any one of claims 1 to 6 wherein the protease is stromelysin and wherein the oligopeptidic sequence comprises or consists of -Pro-TyrAla-Tyr-Trp-Met-Arg-.

13. A wound dressing according to any one of the preceding claims wherein the therapeutic agent is an antimicrobial agent, a pain relieving agent, an antiseptic, an analgesic, a local anaesthetic, or a protease inhibitor.

14. A wound dressing according to any one of the preceding claims wherein the therapeutic agent is incorporated within the matrix.

15. A wound dressing according to claim 14 wherein the wound contacting layer of the dressing may comprise or consist of the matrix within which the therapeutic agent is incorporated.

16. A wound dressing according to claim 15 wherein the dressing comprises a liquid permeable wound contacting layer, an intermediate layer comprising or consisting of the matrix within which the therapeutic agent is incorporated and an outer, liquid-impervious backing layer.

17. A wound dressing according to any one of claims 1 to 13 wherein the dressing comprises a barrier layer which comprises the matrix, the barrier layer being for initially separating the therapeutic agent in the wound dressing from wound fluid when in use.

18. A wound dressing according to claim 17 wherein the barrier layer comprises an apertured sheet having a composition comprising the cross-linked polymers applied thereto in occlusive fashion.

19. A wound dressing according to claim 17 or 18, wherein a layer of the therapeutic substance is provided behind the barrier layer.

20. A wound dressing according to claim 19, wherein an absorbent layer is provided behind the barrier layer and the therapeutic substance is dispersed in the absorbent layer.

21. A wound dressing according to claim 17, wherein the barrier layer substantially encapsulates the therapeutic substance.

22. A wound dressing according to any one of the preceding claims wherein the wound dressing comprises an absorbent layer and/or a backing layer.

## Patentansprüche

1. Wundverband, der ein therapeutisches Mittel und eine Matrix umfasst, die durch Querverbindungen verbundene Polymere umfasst, wobei die Querverbindungen Oligopeptidsequenzen umfassen oder daraus bestehen, die durch eine Protease, die mit Wundflüssigkeit im Zusammenhang steht, spaltbar ist, derart, dass die Rate der Freisetzung des therapeutischen Mittels in Gegenwart der Protease zunimmt.

2. Wundverband nach Anspruch 1, wobei die Protease mit einer Wundinfektion oder einer Ulkusbildung im Zusammenhang steht.

3. Wundverband nach Anspruch 1 oder 2, wobei die Polymere selbst durch die Protease oder andere Faktoren, die in der Wundumgebung vorhanden sein können, nicht abgebaut werden.

4. Wundverband nach Anspruch 1, 2 oder 3, wobei das Polymer ein synthetisches Polymer ist.

5. Wundverband nach Anspruch 4, wobei das Polymer ein Polymer von N-(2-Hydroxypropyl)-methacrylamid (HPMA) ist.

6. Wundverband nach einem der vorangehenden Ansprüche, wobei die Oligopeptidsequenzen aus 3 bis 15 Aminosäuren bestehen.

7. Wundverband nach einem der vorangehenden Ansprüche, wobei die Protease Elastase ist, und wobei die Oligopeptidsequenz lys-gly-ala-ala-ala-lys, -Ala-Ala-Ala, Ala-Ala-Pro-Val, Ala-Ala-Pro-Leu, Ala-Ala-Pro-Phe, Ala-Ala-Pro-Ala oder Ala-Tyr-Leu-Val umfasst oder daraus besteht.

8. Wundverband nach einem der Ansprüche 1 bis 6, wobei die Protease eine Matrix-Metalloproteinase ist, und wobei die Oligopeptidsequenz -Gly-Pro-Y-Gly-Pro-Z-,-Gly-Pro-Leu-Gly-Pro-Z-, -Gly-Pro-Ile-Gly-Pro-Z- oder Ala-Pro-Gly-Leu-Z-, wobei Y und Z Aminosäuren sind, umfasst oder daraus besteht.

9. Wundverband nach einem der Ansprüche 1 bis 6, wobei die Protease eine Kollagenase ist, und wobei die Oligopeptidsequenz -Pro-Leu-Gly-Pro-D-Arg-Z-, -Pro-Leu-Gly-Leu-Leu-Gly-Z-, -Pro-Gln-Gly-Ile-Ala-Gly-Trp-, -Pro-Leu-Gly-Cys(Me)-His-, -Pro-Leu-Gly-Leu-Trp-Ala-, Pro-Leu-Ala-Leu-Trp-Ala-Arg- oder -Pro-Leu-Ala-Tyr-Trp-Ala-Arg-, wobei Z eine Aminosäure ist, umfasst oder daraus besteht.

10. Wundverband nach einem der Ansprüche 1 bis 6, wobei die Protease eine Gelatinase ist, und wobei die Oligopeptidsequenz -Pro-Leu-Gly-Met-Trp-Ser-Arg- umfasst oder daraus besteht.

11. Wundverband nach einem der Ansprüche 1 bis 6, wobei die Protease Thrombin ist, und wobei die Oligopeptidsequenz -Gly-Arg-Gly-Asp-, -Gly-Gly-Arg-, Gly-Arg-Gly-Asp-Ans-Pro-, -Gly-Arg-Gly-Asp-Ser-, -Gly-Arg-Gly-Asp-Ser-Pro-Lys-, -Gly-Pro-Arg-, Val-Pro-Arg- oder -Phe-Val-Arg umfasst oder daraus besteht.

12. Wundverband nach einem der Ansprüche 1 bis 6, wobei die Protease Stromelysin ist, und wobei die Oligopeptidsequenz -Pro-Tyr-Ala-Tyr-Trp-Met-Arg- umfasst oder daraus besteht.

13. Wundverband nach einem der vorangehenden Ansprüche, wobei das therapeutische Mittel ein antimikrobielles Mittel, ein schmerzlinderndes Mittel, ein Antiseptikum, ein Analgetikum, ein lokales Anästhetikum oder ein Proteaseinhibitor ist.

14. Wundverband nach einem der vorangehenden Ansprüche, wobei das therapeutische Mittel in die Matrix aufgenommen ist.

15. Wundverband nach Anspruch 14, wobei die mit der Wunde in Kontakt tretende Schicht des Verbands die Matrix, in die das therapeutische Mittel aufgenommen ist, umfassen oder daraus bestehen kann.

16. Wundverband nach Anspruch 15, wobei der Verband eine flüssigkeitsdurchlässige, mit der Wunde in Kontakt tretende Schicht, eine Zwischenschicht, welche die Matrix, in die das therapeutische Mittel aufgenommen ist, umfasst oder daraus besteht, und eine äußere flüssigkeitsundurchlässige Deckschicht umfasst.

17. Wundverband nach einem der Ansprüche 1 bis 13, wobei der Verband eine Sperrschicht umfasst, welche die Matrix umfasst, wobei die Sperrschicht dafür vorgesehen ist, anfänglich das therapeutische Mittel in dem Wundverband bei Gebrauch von der Wundflüssigkeit zu trennen.

18. Wundverband nach Anspruch 17, wobei die Sperrschicht ein offenes Blatt mit einer Zusammensetzung, welche die quervernetzten Polymere umfasst und auf eine verschließende Weise darauf aufgebracht ist, umfasst.

19. Wundverband nach Anspruch 17 oder 18, wobei eine Schicht der therapeutischen Substanz hinter der Sperrschicht verfügbar gemacht wird.

20. Wundverband nach Anspruch 19, wobei eine absorbierende Schicht hinter der Sperrschicht verfügbar gemacht wird und die therapeutische Substanz in der absorbierenden Schicht dispergiert ist.

21. Wundverband nach Anspruch 17, wobei die Sperrschicht im Wesentlichen die therapeutische Substanz einkapselt.

22. Wundverband nach einem der vorangehenden Ansprüche, wobei der Wundverband eine absorbierende Schicht und/oder eine Deckschicht umfasst.

## Revendications

1. Pansement comprenant un agent thérapeutique et une matrice comprenant des polymères liés par réticulations, lesquelles réticulations comprennent ou sont constituées de séquences oligopeptidiques qui peuvent être clivées par une protéase associée au fluide de la plaie de sorte que la vitesse de libération de l'agent thérapeutique augmente en présente de la protéase.

2. Pansement selon la revendication 1, dans lequel la protéase est associée à une infection de la plaie ou à la formation d'ulcère.

3. Pansement selon la revendication 1 ou 2, dans lequel les polymères eux-mêmes ne sont pas dégradés par la protéase ou d'autres facteurs qui peuvent être présents dans l'environnement de la plaie.

4. Pansement selon la revendication 1, 2 ou 3, dans lequel le polymère est un polymère synthétique.

5. Pansement selon la revendication 4, dans lequel le polymère est un polymère de N-(2-hydroxypropyl)méthyl-acrylamide (HPMA).

6. Pansement selon l'une quelconque des revendications précédentes, dans lequel les séquences oligopeptidiques sont constituées de 3 à 15 acides aminés.

7. Pansement selon l'une quelconque des revendications précédentes, dans lequel la protéase est l'élastase et dans lequel la séquence oligopeptidique comprend ou est constituée de lys-gly-ala-ala-ala-lys-ala-ala-ala, Ala-Ala-Pro-Val, Ala-Ala-Pro-Leu, Ala-Ala-Pro-Phe, Ala-Ala-Pro-Ala ou Ala-Tyr-Leu-Val.

8. Pansement selon l'une quelconque des revendications 1 à 6, dans lequel la protéase est une métalloprotéinase matricielle et dans lequel la séquence oligopeptidique comprend ou est constituée de -Gly-Pro-Y-Gly-Pro-Z, -Gly-Pro-Leu-Gly-Pro-Z, -Gly-Pro-Ile-Gly-Pro-Z, ou Ala-Pro-Gly-Leu-Z-, où Y et Z sont des acides aminés.

9. Pansement selon l'une quelconque des revendications 1 à 6, dans lequel la protéase est une collagénase et dans lequel la séquence oligopeptidique comprend ou est constituée de -Pro-Leu-Gly-Pro-D-Arg-Z-, -Pro-Leu-Gly-leu-Leu-Gly-Z-, -Pro-Gln-Gly-Ile-Ala-Gly-Trp-, -Pro-Leu-Gly-Cys(Me)-His-, -Pro-Leu-Gly-Leu-Trp-Ala-, -Pro-Leu-Ala-Leu-Trp-Ala-Arg-, ou Pro-Leu-Ala-Tyr-Trp-Ala-Arg-, où Z est un acide aminé.

10. Pansement selon l'une quelconque des revendications 1 à 6, dans lequel la protéase est une gélatinase et dans lequel la séquence oligopeptidique comprend ou est constituée de -Pro-Leu-Gly-Met-Trp-Ser-Arg-.

11. Pansement selon l'une quelconque des revendications 1 à 6, dans lequel la protéase est la thrombine et dans lequel la séquence oligopeptidique comprend ou est constituée de -Gly-Arg-Gly-Asp-, -Gly-Gly-Arg-, -Gly-Arg-Gly-Asp-Asn-Pro-, -Gly-Arg-Gly-Asp-Ser-, -Gly-Arg-Gly-Asp-Ser-Pro-Lys-, -Gly-Pro-Arg-,-Val-Pro-Arg-, ou Phe-Val-Arg-.

12. Pansement selon l'une quelconque des revendications 1 à 6, dans lequel la protéase est la stromélysine et dans lequel la séquence oligopeptidique comprend ou est constituée de -Pro-Tyr-Ala-Tyr-Trp-Met-Arg-.

13. Pansement selon l'une quelconque des revendications précédentes, dans lequel l'agent thérapeutique est un agent antimicrobien, un agent soulageant une douleur, un antiseptique, un analgésique, un anesthésique local, ou un inhibiteur de la protéase.

14. Pansement selon l'une quelconque des revendications précédentes, dans lequel l'agent thérapeutique est incorporé dans la matrice.

15. Pansement selon la revendication 14, dans lequel la couche du pansement en contact avec la plaie peut comprendre ou être constituée de la matrice dans laquelle l'agent thérapeutique est incorporé.

16. Pansement selon la revendication 15, dans lequel le pansement comprend une couche en contact avec la plaie perméable à un liquide, une couche intermédiaire comprenant ou constituée de la matrice dans laquelle l'agent thérapeutique est incorporé et une couche de support, externe, imperméable à un liquide.

17. Pansement selon l'une quelconque des revendications 1 à 13, dans lequel le pansement comprend une couche barrière qui comprend la matrice, la couche barrière étant utilisée pour séparer initialement l'agent thérapeutique dans le pansement du fluide de la plaie lors de son utilisation.

18. Pansement selon la revendication 17, dans lequel la couche barrière comprend une feuille trouée ayant une composition comprenant les polymères réticulés appliqués sur celle-ci de manière occlusive.

19. Pansement selon la revendication 17 ou 18, dans lequel on propose une couche de la substance thérapeutique derrière la couche barrière.

20. Pansement selon la revendication 19, dans lequel on propose une couche absorbante derrière la couche barrière et la substance thérapeutique est dispersée dans la couche absorbante.

21. Pansement selon la revendication 17, dans lequel la couche barrière encapsule essentiellement la substance thérapeutique.

22. Pansement selon l'une quelconque des revendications précédentes, dans lequel le pansement comprend une couche absorbante et/ou une couche de support.
